# EUROPEAN PATENT APPLICATION

(11) **EP 4 745 576 A1**
(43) Date of publication of application: **20.05.2026**
(21) Application number: 24383255.7
(22) Date of filing: 19.11.2024
(51) Int. Cl.: G01N 33/66, G01N 33/68

(54) **USE OF N-GLYCOME FOR PREDICTING THE RISK OF SUFFERING FROM TYPE 2 DIABETES**

(71) Applicant: Fundación Pública Galega Instituto de Investigación Sanitaria de Santiago de Compostela, 15706 Santiago de Compostela A Coruña (ES); Servizo Galego De Saúde, 15703 Santiago de Compostela, A Coruna (ES); Universidade de Santiago de Compostela (USC), 15872 Santiago de Compostela A Coruña (ES)
(72) Inventor: CARBALLO FERNÁNDEZ, Iago, 15706 Santiago de Compostela (ES); GONZÁLEZ QUINTELA, Arturo, 15706 Santiago de Compostela (ES); LADO BALEATO, Óscar, 15706 Santiago de Compostela (ES); GUDE SAMPEDRO, Francisco, 15706 Santiago de Compostela (ES)
(74) Representative: Hoffmann Eitle

(57) **Abstract**

The present invention refers to a method for predicting the risk of suffering from type 2 diabetes which comprises assessing the presence of N-glycome peak 37 and N-glycome peak 24 in a biological sample obtained from the subject.

## Description

### FIELD OF THE INVENTION

The present invention refers to the medical field. Particularly, the present invention refers to a method for predicting the risk of suffering from type 2 diabetes which comprises assessing the abundance of N-glycome peak 37 and N-glycome peak 24 in a biological sample obtained from the subject.

### STATE OF THE ART

Type 2 diabetes constitutes the majority of diabetes cases worldwide, and its multifactorial etiology culminates in insulin being unable to perform its function. Prediabetes is a previous stage of type 2 diabetes, characterized by blood glucose levels that are high but lower than the diabetes threshold. Annually, approximately 5%-10% of patients with prediabetes progress to diabetes, and a similar percentage return to normoglycemia. A number of normoglycemic patients progress to diabetes very quickly, without remaining long in a prediabetic state. Conversely, a number of prediabetic conditions persist without progressing to diabetes for many years and can even return to normoglycemia.

Numerous environmental, genetic, and metabolomic factors have been identified as risk factors for developing type 2 diabetes. Various prediction models for incident prediabetes and type 2 diabetes have been developed, as well as lifestyle and drug recommendations for reducing the risk of progression. However, the best screening tools are complex, and type 2 diabetes in certain patients remains undetectable. Recent glycomic studies have shown that total plasma/serum N-glycome (TPSNG) differs between people with diabetes and normoglycemic individuals. Moreover, total plasma N-glycome (TPNG) might predict the risk of developing type 2 diabetes and to even improve one of the best risk scores.

Glycomics is the comprehensive study of all glycan structures, function, generation, modification, degradation, and regulation of a given cell type or organism. Glycosylation refers to a complex and organized biological process involving numerous genes, transcription factors, and signaling pathways, leading to the binding of a glycan to another glycan, protein, or lipid. Glycosylation participates in co-translational (*N*-glycosylation) and post-translational (*O-*glycosylation) modifications and differs from glycation, a non-enzymatic reaction. Glycans also cover the surface of cells, comprising the main part of the glycocalyx, which provides an interface with the extracellular environment and is essential in cell-cell communication.

*N*-glycome encompasses all oligosaccharides covalently linked to the amide nitrogen of an asparagine (Asn) residue that belongs to an Asn-X-Ser/Thr consensus sequence of a protein, where X can be any amino acid except proline. It is estimated that most human proteins are glycoproteins, and the large majority of them are *N*-glycosylated. TPSNG analysis provides information about the levels and glycosylation patterns of plasma/serum proteins and has revealed interesting discoveries in the last decade. Specific glycans and disease patterns of glycosylation (signatures) can be useful for the diagnosis, prognosis, and management of type 2 diabetes.

So, there is an unmet medical need of finding reliable method aimed at predicting the risk of suffering from type 2 diabetes. The present invention is focused on solving this problem by identifying predictive biomarkers of the incidence of type 2 diabetes in the total serum N-glycome (TSNG) of a general adult population, and to explore its usefulness as a prognostic tool alone and in combination with classical prediction factors.

### DESCRIPTION OF THE INVENTION

### Brief description of the invention

The present invention refers to a method for predicting the risk of suffering from type 2 diabetes which comprises assessing the abundance of N-glycome peak 37 and N-glycome peak 24 in a biological sample obtained from the subject.

Glycosylation is a tightly controlled co-translational and post-translational enzymatic modification. Total N-glycome profiling in blood serum/plasma provides information on the most common serum/plasma glycosylation. So, the ability of total serum N-glycome to predict this disease is herein explored in a general adult population.

The present invention is based on a prospective cohort study included a random sample of 1516 adults from a single Spanish municipality. Participants' glycemic status (non-diabetes, type 2 diabetes) was evaluated at baseline and at a mean follow-up of 7.4 years. Total serum N-glycome at baseline was also measured. Serum enzymatic N-glycan release was performed on a robotic platform followed by HILIC-UPLC glycan separation. Total serum N-glycans were quantified and employed alone, as well as in combination with classical risk factors, to construct type 2 diabetes prediction models.

In a nutshell, the inventors of the present invention concluded that total serum N-glycome peak 37, mainly composed of triantennerary glycans containing outer arm fucose (A3F1G3S[3,3,3]3 and A3F1G3S[3,3,6]), predisposed participants to type 2 diabetes; however, total serum N-glycome peak 24, mainly composed of disialylated glycans (A2G2S[3,6]2 and A2G2S[3,3]2), was protective against type 2 diabetes. Of note, the interaction between total serum N-glycome peaks 37 and 24 predicted the incidence of type 2 diabetes over time (area under the curve 0.801 [0.750-0.853]). Their predictive power had an independent and additive effect on classical prediction factors. The ratio between total serum N-glycome peaks 37 and 24 has an independent and additive effect on predictive power of classical prediction factors (HbAlc, age, sex, BMI, HOMA-IR, TNF-alpha).

In conclusion, the ratio or interaction between total serum N-glycome peaks 37 and 24 constitutes a predictive signature for type 2 diabetes improving the classical prediction tools.

So, the first embodiment of the present invention refers to an *in vitro* method for predicting the risk of suffering from type 2 diabetes in a subject, wherein the method comprises assessing the abundance of N-glycome peak 37 and N-glycome peak 24 in a biological sample obtained from the subject.

The second embodiment of the present invention refers to the *in vitro* use of N-glycome peak 37 and N-glycome peak 24, or of a kit comprising reagents for assessing the abundance of N-glycome peak 37 and N-glycome peak 24, in a biological sample obtained from the subject, for predicting the risk of suffering from type 2 diabetes.

In a preferred embodiment of the present invention, higher abundance of N-glycome peak 37 (with respect to a pre-established threshold value) is an indication of a higher risk of suffering from type 2 diabetes and/or higher abundance of N-glycome peak 24 (with respect to a pre-established threshold value) is an indication of a lower risk of suffering from type 2 diabetes.

In a preferred embodiment, the present invention comprises assessing the ration between N-glycome peak 37 and N-glycome peak 24 for predicting the incidence of type 2 diabetes.

In a preferred embodiment of the present invention, the biological sample is selected from blood, plasma or serum.

In a preferred embodiment of the present invention, the biological sample is serum.

In a preferred embodiment of the present invention, the N-glycome peak 37 comprises triantennerary glycans containing outer arm fucose.

In a preferred embodiment of the present invention, the N-glycome peak 37 comprises the glycans: A3F1G3S[3,3,3]3, A3F1G3S[3,3,6]3, FA3BG3S[3,3,3]3, A3G3S[3,3,6]3, *FA3BG3S[6,6,6]3, *A3G3S[3,6,6]3 and/or *A3G3S[3,3,3]3.

In a preferred embodiment of the present invention, the N-glycome peak 24, comprises disialylated glycans.

In a preferred embodiment of the present invention, the N-glycome peak 24, comprises the glycans: *A2G2S[3,6]2, *A2G2S[3,3]2, *FA2G2S[3,3]2, A2F1G2S[3]1 and/or A2F1G2S[6]1.

In a preferred embodiment of the present invention, the N-glycans are separated and quantified using a quantitative analytical technique.

In a preferred embodiment of the present invention, the abundance of N-glycome peak 37 and N-glycome peak 24 is assessed by chromatography.

In a preferred embodiment of the present invention, the abundance of N-glycome peak 37 and N-glycome peak 24 is assessed by ultra-performance liquid chromatography (UPLC), capillary electrophoresis (CE), MALDI-TOF mass spectrometry, liquid chromatography coupled with mass spectrometry (LC-MS).

In a preferred embodiment of the present invention, the abundance of N-glycome peak 37 and N-glycome peak 24 is assessed by ultra-performance liquid chromatography (UPLC).

The present invention also refers to:
A method for treating patient who may be suffering from prediabetes or type 2 diabetes, wherein the method comprises a previous step of predicting the risk of suffering from type 2 diabetes by assessing, according to the present invention, the abundance of N-glycome peak 37 and N-glycome peak 24 in a biological sample obtained from the subject. The therapy may comprise any treatment known in the prior art to treat prediabetes or type 2 diabetes like, for instance: Metformin (often the first-line medication, it helps lower glucose production in the liver and improves insulin sensitivity), Sulfonylureas (Stimulate the pancreas to release more insulin), DPP-4 Inhibitors (they help reduce blood sugar levels without causing weight gain), GLP-1 Receptor Agonists (they enhance insulin secretion and suppress glucagon, often leading to weight loss), SGLT2 Inhibitors (they help the kidneys remove excess glucose from the body through urine) or insulin therapy (it may be needed if blood sugar levels are not controlled with oral medications alone).

In a preferred embodiment, the present invention is a computer-implemented invention, wherein a processing unit (hardware) and a software are configured to: a) Receive N-glycome peak 37 and N-glycome peak 24 values, b) process the values received for finding substantial variations or deviations, and c) provide an output through a terminal display of the variation or deviation of the value, wherein the variation or deviation of the value indicates the risk of suffering from type 2 diabetes.

For the purpose of the present invention the following terms and abbreviations are defined:
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.
- "N-glycome" refers to the complete set of N-linked glycans (sugar chains) attached to proteins within a cell, tissue, or organism. N-glycans are a type of carbohydrate chain that is attached to the nitrogen atom (N) in the side chain of an asparagine amino acid residue in a protein. This process, known as N-glycosylation, is critical for the proper folding, stability, and function of many proteins. The N-glycome is important in various biological processes, including cell-cell communication, immune response, and protein trafficking. Alterations in the N-glycome can be associated with diseases, making it a significant area of study in glycomics and biomedicine. N-glycans are herein identified using Oxford type or Oxford method nomenclature such as it is identified by this reference *[*Harvey DJ, Merry AH, Royle L, Campbell MP, Dwek RA, Rudd PM. Proposal for a standard system for drawing structural diagrams of N- and O-linked carbohydrates and related compounds. Proteomics. 2009;9(15):3796-801. doi: 10.1002/pmic.200900096*.* Erratum in: Proteomics. 2009;9(21):5002*].*
- According to the present invention, a reference value can be a "pre-established threshold value" or a "cut-off" value. Typically, a "threshold value" or "cut-off value" can be determined experimentally, empirically, or theoretically. Thus, for instance, the subject is likely to suffer from a higher mortality risk when an increased abundance of the claimed N-glycome is identified, as compared with a pre-established "threshold value". A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.

### • Abbreviations:

- AEGIS: A-Estrada Glycation and Inflammation Study
- Asn: Asparagine
- B: Balance
- BMI: Body mass index
- CRP: C-reactive protein
- DPP-4: Dipeptidyl peptidase-4
- FPG: Fasting plasma glucose
- GLP-1: Glucagon like peptide 1
- GP: Glycome peak
- HbAlc: Glycated hemoglobin
- HILIC: Hydrophilic interaction chromatography
- HOMA-IR: Homeostasis model assessment of insulin resistance
- ilr: isometric log-ratio
- ROC: Receiver operating characteristic
- Ser: Serine
- SGLT2: Sodium-glucose cotransporter-2
- te: (non-linear interaction) term
- Thr: Threonine
- TNF: Tumor necrosis factor
- TPNG: Total plasma N-glycome
- TPSNG: Total plasma/serum N-glycome
- TSNG: Total serum N-glycome
- UPLC: Ultra-performance liquid chromatography

### Brief description of the figures

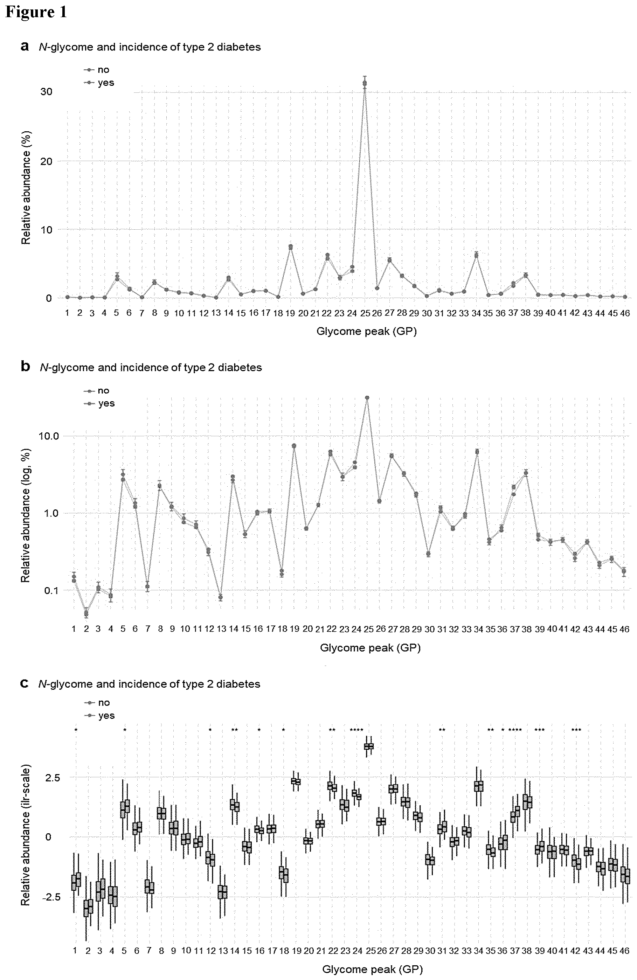

**Figure 1****.** Difference in total serum N-glycome profiles between participants who developed type 2 diabetes during the follow-up period and those who did not. Panel a displays the average total serum N-glycome profiles stratified by incidence of type 2 diabetes, while Panel b displays the same data but in a logarithmic scale, and Panel c in an ilr-scale. Abbreviations: ilr, isometric log-ratio. *P <0.10; **P <0.05; ***P <0.001; ****P<0.0001.

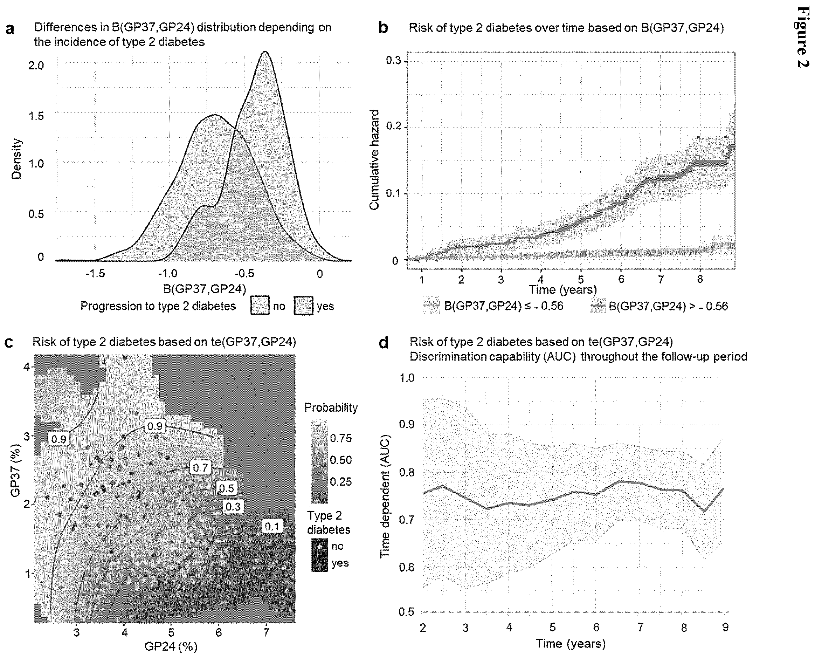

**Figure 2****.** Association of glycome peaks 37 and 24 with the incidence of type 2 diabetes. Panel a depicts the distribution of the B(GP37,GP24) for participants who developed type 2 diabetes and those who did not. Panel b shows the cumulative incidence of type 2 diabetes stratified by B(GP37,GP24). Panel c represents the effect of GP37 and GP24 on the incidence of type 2 diabetes using a non-linear interaction term [te(GP37,GP24)]. Panel d shows the discrimination capability of GP37 and GP24 [te(GP37,GP24)] for the incidence of type 2 diabetes throughout the follow-up period. Abbreviations: B, balance; GP, glycome peak; te, (non-linear interaction) term.

### Detailed description of the invention

The present invention is illustrated by means of the Examples set below without the intention of limiting its scope of protection.

### Example 1. Material and methods

### Example 1.1. Study design and data sources

The A-Estrada Glycation and Inflammation Study (AEGIS) is a prospective cohort study developed in A-Estrada, a municipality in Northwestern Spain. An outline of the study is available at www.clinicaltrials.gov (code NCT01796184). When the study began in 2012, A-Estrada had an adult population (age ≥18 years) of 18,474. An age-stratified random sample of 3500 adult individuals was drawn from Spain's National Health System Registry, which covers more than 95% of the population. From the initial 3500 individuals, 2230 could be assessed for eligibility and displayed no exclusion criteria (no health care, moved away, died, no response to communication attempts); of these, 1516 individuals agreed to participate (overall participation rate, 68%). Participation was higher among women than men (71% vs. 65%), and there were no significant differences in terms of age or residence (rural vs. urban) between participants and non-participants.

From November 2012 through March 2015, all participants were successively contacted and asked to attend the Primary Care Center for evaluation through a clinical interview that included a structured questionnaire, physical examination, and fasting venous blood sampling (n=1516, 44.7% men, aged 18-91 years, median 52 years). All participants were again evaluated by a physician who checked computerized medical records until July-August 2022, which included all blood tests performed and drugs prescribed at least since 2008, as well as primary and hospital medical care. Participants diagnosed with type 2 diabetes in the medical records or with fasting plasma glucose (FPG) >125 mg/dL in 2 consecutive tests or glycated hemoglobin (HbAlc) levels ≥6.5% (48 mmol/mol) in 2 consecutive tests or both FPG >125 mg/dL and HbAlc ≥6.5% (48 mmol/mol) in the same test or in consecutive tests were classified as having diabetes. Participants with previous diabetes or diabetes onset other than type 2 diabetes were excluded from the data analyses. Transient hyperglycemia in the diabetic range occurring during a severe or pancreatic illness or due to drug (corticoid) intake was not considered a diagnosis of diabetes. The timepoints of evaluation were the date of study recruitment (November 2012-March 2015) and the date of the last blood test available when the computerized medical records were consulted (July 2022-August 2022). The date of type 2 diabetes diagnosis was collected in all cases.

### Example 1.2. Ethical Issues

All participants provided written informed consent. The general survey and the specific glycomic studies were approved by the Galician Regional Ethics Committee (codes 2010-315 and 2016-464, respectively) and conformed to the current Helsinki Declaration.

### Example 1.3. Assessment of metabolic abnormalities

Participants were considered as having metabolic syndrome if they had at least 3 of the following Adult Treatment Panel III criteria: 1) abdominal obesity; 2) hypertriglyceridemia; 3) low levels of high-density lipoprotein cholesterol; 4) increased blood pressure; and 5) hyperglycemia.

### Example 1.4. Assessment of smoking and alcohol consumption

Consumers of at least 1 cigarette per day were classified as smokers. Individuals who had quit smoking during the preceding year were still considered smokers.

Alcohol consumption was evaluated in standard drinking units by summing the number of glasses of wine (1 unit, ~10 g), bottles of beer (1 unit, ~10 g), and spirits (2 units, ~20 g) regularly consumed per week. Individuals with an alcohol consumption of 1-13 units/week were considered as light drinkers, those with 14-27 units/week as moderate drinkers, and those with ≥28 units/week as heavy drinkers.

### Example 1.5. Assessment of physical activity

All study participants completed the short version of the International Physical Activity Questionnaire (freely available at https://sites.google.com/site/theipaq/home), which has been validated in Spain. The questionnaire allows for the calculation of metabolic equivalents of various tasks and for stratification of habitual physical activity as low, moderate, or high.

### Example 1.6. Determination of fasting plasma glucose

Glucose levels were determined in fresh serum samples from fasting participants by the glucose oxidase method in an ADVIA 2400 Clinical Chemistry System (Siemens, Germany).

### Example 1.7. Determination of glycated hemoglobin

Glycated hemoglobin (HbAlc) levels were determined in fresh serum samples by high-performance liquid chromatography in an ADAMS Aic HA-8160 analyzer (ARKRAY, Japan); all HbAlc values were converted to Diabetes Control and Complications Trial (DCCT)-aligned values.

### Example 1.8. Estimation of insulin resistance

Insulin resistance was estimated using the Homeostasis Model Assessment of Insulin Resistance (HOMA-IR) as the fasting serum glucose (mg/dL) × fasting serum insulin (µU/mL) ÷ 405.23.

### Example 1.9. Insulin assay

Insulin concentrations were measured in fresh serum samples from fasting participants using a commercial chemiluminescent immunoassay in an ADVIA Centaur XP Immunoassay System (Siemens, Germany).

### Example 1.10. C-reactive protein assay

Wide-range C-reactive protein (CRP) concentrations were measured in fresh serum samples using commercial latex-enhanced immunoturbidimetry in an ADVIA 2400 Clinical Chemistry System (Siemens, Germany).

### Example 1.11. Tumor necrosis factor-alpha assay

Tumor necrosis factor (TNF)-alpha concentrations were measured in fresh samples using a commercial chemiluminescent immunoassay in an IMMULITE 1000 System (Siemens, Germany).

### Example 1.12. Serum N-glycan analyses

The complete procedure is disclosed in *[*O'Flaherty R, Simon Á, Alonso-Sampedro M, Sánchez-Batán S, Fernández-Merino C, Gude F, et al (2022) Changes in serum N-glycome for risk drinkers: a comparison with standard markers for alcohol abuse in men and women. Biomolecules 12(2):241. https://doi.org/10.3390/biom12020241]. *N*-glycans were profiled by a modified high-throughput automated method from 5 µL of serum samples previously stored at -80°C for further use [Stöckmann H, O'Flaherty R, Adamczyk B, Saldova R, Rudd PM (2015) Automated, high-throughput serum glycoprofiling platform. Integr Biol 7(9):1026-1032. https://doi.org/10.1039/c5ib00130g]*.* Briefly, the samples were denatured and *N-*glycans were enzymatically released from the protein backbone via peptide:*N-*glycosidase F. The glycans were then immobilized on solid supported hydrazide beads, and excess reagents were removed by centrifuge filtration. The glycans were released from the solid support and labeled with fluorophore 2-aminobenzamide.

Hydrophilic interaction chromatography (HILIC) ultra-performance liquid chromatography (UPLC) was performed, assigning glucose unit values from retention times. The chromatograms were all separated in the same manner into 46 peaks according to Saldova et al. [Saldova R, Asadi Shehni A, Haakensen VD, Steinfeld I, Hilliard M, Kifer I, et al (2014) Association of N-glycosylation with breast carcinoma and systemic features using high-resolution quantitative UPLC. J Proteome Res 13(5):2314-2327*. https:*//*doi.org*/*10.1021*/*pr401092y*], and the amount of glycans in each peak was expressed as a percentage of the total integrated area. Glycan structures were annotated using the symbol nomenclature for glycans and DrawGlycan-SNFG software [Cheng K, Zhou Y, Neelamegham S (2016) DrawGlycan-SNFG: a robust tool to render glycans and glycopeptides with fragmentation information. Glycobiology 27(3):200-205. https://%doi.org/10.1093/glycob/cww115], *[*Nee7amegham S, Aoki-Kinoshita K, Bolton E, Frank M, Lisacek F, Lütteke T, et al (2019) Updates to the symbol nomenclature for glycans guidelines. Glycobiology 29(9):620-624. https://doi.org/10.1093/glycob/cwz045*],* with the assistance of GlycoStore.org (accessed on 11 November 2021) [Zhao S, Walsh I, Abrahams J, Royle L, Nguyen-Khuong T, Spencer D, et al. GlycoStore: a database of retention properties for glycan analysis. Bioinformatics 2018;34:3231-3232].

A summary of glycome peaks (GPs) and the corresponding main A-glycan structures are shown in [O'Flaherty R, Simon A, Alonso-Sampedro M, Sánchez-Batán S, Fernández-Merino C, Gude F, et al (2022) Changes in serum N-glycome for risk drinkers: a comparison with standard markers for alcohol abuse in men and women. Biomolecules 12(2):241. https://doi.org/10.3390/biom12020241]*.* Groups of GPs were defined from their common features, as follows [Saldova R, Asadi Shehni A, Haakensen VD, Steinfeld I, HilliardM, Kifer I, et al (2014) Association of N-glycosylation with breast carcinoma and systemic features using high-resolution quantitative UPLC. J Proteome Res 13(5):2314-2327. https://doi.org/10.1021/pr401092y]:
- Sialylation: S0 (neutral, GP1-15); S1 (monosialylated, GP16-23 + GP30); S2 (disialylated, GP24-29 + 31); S3 (trisialylated, GP32-40); and S4 (tetrasialylated, GP41-46).
- Galactosylation: G0 (agalactosylated, GP1-2 + GP4-5 + GP6/2 + GP12/2); G1 (monogalactosylated, GP3 + GP7-10 + GP12/2 + GP16-18 + GP21/2); G2 (digalactosylated, GP13-15 + GP19-20 + GP21/2 + GP22-28); G3 (trigalactosylated, GP29 + GP31-37); and G4 (tetragalactosylated, GP30 + GP38-46).
- Branching: A1 (monoantennary, GP1-3 + GP12/2 + GP21/2); A2 (biantennary, GP4-5 + GP6/2 + GP7-10 + GP12/2 + GP13-20 + GP21/2 + GP22-28); A3 (triantennary, GP29 + GP31-37); and A4 (tetra-antennary, GP30 + GP38-46).
- Oligomannose: GP6/2 + GP11.
- Fucosylation: Core-fucose (GP2 + GP5 + GP6/2 + GP8-10 + GP14-15 + GP17-18 + GP22-23 + GP27-28 + GP36 + GP44/2); and outer-arm fucose (GP37 + GP40 + GP41/3 + GP45 + GP46/3).

In addition, mass spectrometry-assisted glycan characterization was performed for 2 representative samples and a technical replicate. Otherwise, the major glycans were identified and assigned based on their glucose unit values cross-referenced in Glycobase, now migrated to Glycostore and based on previous assignments in Saldova et al. *[*Saldova R, Asadi Shehni A, Haakensen VD, Steinfeld I, Hilliard M, Kifer I, et al (2014) Association of N-glycosylation with breast carcinoma and systemic features using high-resolution quantitative UPLC. J Proteome Res 13(5):2314-2327. https://doi.org/10.1021/pr401092y]*.*

A summary of *N*-glycan structures identified by mass spectrometry and their correlation with *N*-glycan structures identified by HILIC-UPLC are shown in *[*O'Flaherty R, Simon Á, Alonso-Sampedro M, Sánchez-Batán S, Fernández-Merino C, Gude F, et al (2022) Changes in serum N-glycome for risk drinkers: a comparison with standard markers for alcohol abuse in men and women. Biomolecules 12(2):241. https://doi.org/10.3390/biom12020241].

### Example 1.13. Statistical Analysis

Wilcoxon and chi-squared tests were applied to check for differences in continuous and categorical variables, respectively, in the participant groups.

*N*-glycome data were analyzed following compositional data analysis techniques. Differences between patient groups were analyzed applying a two-sample test for high-dimensional compositional data *[*Cao 7, Lin W, Li H (2018) Two-sample tests of high-dimensional means for compositional data. Biometrika 105(1): 115 -132. https://doi.org/10.1093/biomet/asx060]. To complete this multivariate test, several non-parametric Mann-Whitney U tests were applied on the ilr-transformed values to explore individual peak differences. P-values were corrected by Bonferroni.

The selbal algorithm *[*Rivera-Pinto J, Egozcue JJ, Pawlowsky-Glahn V, Paredes R, Noguera-Julian M, Calle ML (2018) Balances: a new perspective for microbiome analysis. mSystems 3(4):e00053-18. https://doi.org/10.1128/mSystems.00053-18] was employed to determine the optimal combination of GPs for predicting type 2 diabetes incidence. Unlike multiple logistic regression, this algorithm does not include raw variables in a linear equation in real space. Instead, they are incorporated as part of the so-called "balances" (which are the normalized logarithmic ratios between two disjoint subsets of components from the original data). Each subset can have a different dimension, typically ranging from 1 to 5, resulting in thousands of predictor variables derived from the combination of original GPs. A lasso regression was then employed to identify the most informative balances for predicting participant outcomes. Additionally, we estimated non-linear interactions between *N*-glycome peaks and their association with type 2 diabetes incidence using bivariate thin plate splines *[*Wood SN (2017) Generalized additive models: an introduction with R, second edition. CRC Press, Boca Ratón].

The area under the curve (AUC) from the receiver operating characteristic (ROC) analysis were used to assess the GPs' predictive performance. The ROC curves and the AUC, with 95% confidence intervals, were calculated using the pROC R package *[*Robin X, Turck N, Hainard A, Tiberti N, Lisacek F, Sanchez JC, et al (2011) pROC: an open-source package for R and S+ to analyze and compare ROC curves. BMC Bioinformatics 2011;12: 77. https://doi.org/10.1186/1471-2105-12-77]. Statistical analyses were performed in R [R Core Team (2023) R: a language and environment for statistical computing. R Foundation for Statistical Computing, Vienna. Available from: https://www.R-project.org/. Accesed 2023*],* using the packages' compositions *[*van den Boogaart KG, Tolosana-Delgado R, Bren M (2023) compositions: Compositional Data Analysis . R package version 2.0-5. Available from: https://CRAN.R-project.org/package=compositions. Accesed 2023*],* and mgcv *[*Wood SN (2017) Generalized additive models: an introduction with R, second edition. CRC Press, Boca Ratón]*.*

### Example 2. Results

### Example 2.1. Database description

At recruitment, 1331 participants were non-diabetic and 179 had type 2 diabetes (5 were excluded due to a diagnosis of type 1 diabetes, and 1 was excluded due to steroid-induced diabetes). During follow-up, 71 participants without diabetes developed type 2 diabetes, and 2 participants without diabetes were excluded due to diabetes onset coinciding with pancreatic cancer diagnosis. The type 2 diabetes incidence rate was 5.3% (95% CI 4.2-6.7) after a mean participant follow-up of 7.4 years. Individuals who developed type 2 diabetes after follow-up were older, had a higher BMI, a higher proportion of metabolic syndrome, and lower physical activity. Moreover, they showed higher values of glycemic (FPG, HbAlc) and inflammatory (CRP and TNF-alpha) markers, as well as higher insulin resistance (HOMA-IR) **(Table 1).**

**Table 1. Baseline characteristics of AEGIS participants stratified by final type 2 diabetes status**

| Variable | | AEGIS (n=1516) | No DM (n=1260) | T2D (n=71) | P |
|---|---|---|---|---|---|
| Age (years)* | | 52 [39, 67] | 49 [36, 64] | 60 [51,68] | <0.001 |
| Sex (male)^{†} | | 678 (44.7%) | 545 (43.2%) | 31 (43.7%) | 0.624 |
| BMI (kg/m²)* | | 27.8 [24.6,31.4] | 27.1 [24.1, 30.5] | 32.3 [28.9, 35.2] | <0.001 |
| MetS^{†} | | 314 (20.7%) | 147 (11.7%) | 35 (49.3%) | <0.001 |
| Smoking status^{†} | | | | | 0.069 |
| | - Non | 825 (54.4%) | 682 (54.1%) | 43 (60.6%) | |
| | - Ex | 395 (26.1%) | 311 (24.7%) | 21 (29.6%) | |
| | - Smoker | 296 (19.5%) | 267 (21.2%) | 7 (9.8%) | |
| Alcohol consumption^{†} | | | | | 0.478 |
| | - Abstemious | 546 (36.0%) | 452 (35.9%) | 28 (39.4%) | |
| | - Light drinker | 598 (39.5%) | 517 (41.0%) | 23 (32.4%) | |
| | - Moderate | 241 (15.9%) | 190 (15.1%) | 12 (16.9%) | |
| | - Heavy | 131 (8.6%) | 101 (8.0%) | 8 (11.3%) | |
| Physical activity^{†} | | | | | 0.045 |
| | - Low | 596 (39.3%) | 472 (37.4%) | 37 (52.1%) | |
| | - Medium | 552 (36.4%) | 461 (36.6%) | 21 (29.6%) | |
| | - High | 368 (24.3%) | 327 (26.0%) | 13 (18.3%) | |
| FPG (mg/dL)* | | 89 [82, 100] | 86 [81, 94] | 105 [94, 111] | <0.001 |
| HbAlc (%)* | | 5.4 [5.2, 5.7] | 5.4 [5.2, 5.6] | 5.9 [5.7, 6.1] | <0.001 |
| HbAlc (mmol/mol)* | | 36 [33, 39] | 36 [33, 38] | 41 [39, 43] | <0.001 |
| HOMA-IR (%)* | | 2.4 [1.6, 3.5] | 2.2 [1.5, 3.1] | 3.9 [2.7, 5.7] | <0.001 |
| CRP (mg/dL)* | | 0.14 [0.04, 0.39] | 0.13 [0.04, 0.36] | 0.27 [0.10, 0.66] | <0.001 |
| TNF-alpha (pg/mL)* | | 7.4 [6.1, 9.0] | 7.2 [6.0, 8.7] | 8.3 [7.0, 9.5] | <0.001 |

| | | | | | |
|---|---|---|---|---|---|
| *Abbreviations: AEGIS, A-Estrada Glycation and Inflammation Study; BMI, body mass index; CRP, C-reactive protein; DM, diabetes mellitus; FPG, fasting plasma glucose; HbA1c, glycated hemoglobin; HOMA-IR, Homeostasis Model Assessment of Insulin Resistance; MetS, metabolic syndrome; TNF, tumor necrosis factor; T2D, type 2 diabetes. *Median and [interquartile range], †absolute frequency and (percentage).* | | | | | |

### Example 2.2. Total serum N-glycome for type 2 diabetes prediction

The participants who developed type 2 diabetes showed a greater abundance of GP1, 5, 31, 36, 37, and 39 and a lesser abundance of GP12, 14, 16, 18, 22, 24, 35, and 42 than those who did not develop type 2 diabetes **(****Figure 1****).**

The selbal algorithm for 5 cross-validations and 10 interactions, determined through a multivariate analysis of all GPs, revealed the optimal balance (B) for predicting type 2 diabetes for 2 glycome peaks (GP37 and GP24): B(GP37,GP24), AUC of 0.79 (95% CI 0.74-0.84). Higher relative values of GP37 to GP24 showed a higher risk of type 2 diabetes **(****Figure 2a****).** Specifically, each decimal unit increase of this balance had an odds ratio of 1.55 (95% CI 1.40-1.74). This balance was also associated with type 2 diabetes prevalence, with an odds ratio of 1.25 (95% CI 1.17-1.33) for each decimal unit increase.

A survival proportional hazard model also showed an association between B(GP37,GP24) and type 2 diabetes. The estimated hazard ratio for type 2 diabetes incidence was 1.48 (95% CI 1.34-1.64) for each decimal unit increase in B(GP37,GP24). A cut-off for B(GP37,GP24) >-0.56 displayed a cumulative incidence of 11.7% after 7 years, whereas those with lower values had a cumulative incidence of 1.2% **(****Figure 2b****).**

The non-linear interaction effect between GP37 and GP24, expressed by the term te(GP37,GP24), was explored using a logistic regression model, achieving higher prediction accuracy for type 2 diabetes (AUC 0.80 [95% CI 0.75-0.85]). In this model, unlike the previous one, a greater abundance of GP24 had a protective effect against type 2 diabetes incidence only when GP37 remained low, whereas for high GP37, GP24 had no effect **(****Figure 2c****).**

The predictive accuracy of GP37 and 24 remained stable near 0.80 throughout the follow-up period **(****Figure 2d****).** *N*-glycome is therefore a potential predictor of the incidence of type 2 diabetes, both in the short and long term.

The association between te(GP37,GP24) and classical risk factors (basal glycemic status shown by HbA1c, age, sex, BMI, insulin resistance measured by HOMA-IR, and inflammation status according to TNF-alpha) was studied in the incidence of type 2 diabetes. Metabolic syndrome, physical activity, and CRP were excluded due to high collinearity with the aforementioned risk factors. Four different logistic regression models were built **(Table 2).**

**Table 2. Effect of risk factors on the incidence of type 2 diabetes estimated using logistic regression models for two univariate models (models 1 and 2) and two multivariate models (3 and 4)**

| Predictor variables | Model 1 OR (95% CI) | Model 2 OR (95% CI) | Model 3 OR (95% CI) | Model 4 OR (95% CI) |
|---|---|---|---|---|
| te(GP37,GP24)* | 1.14 (1.09, 1.18) | -- | -- | 1.03 (1.01, 1.05) |
| HbAlc (%)^{†} | -- | 1.52 (1.39, 1.65) | 1.41 (1.28, 1.54) | 1.37 (1.25, 1.51) |
| Age (years) | -- | -- | 1.00 (0.98, 1.02) | 1.00 (0.98, 1.02) |
| Sex (ref: female) | -- | -- | 0.95 (0.54, 1.69) | 1.07 (0.60, 1.94) |
| BMI (kg/m²) | -- | -- | 1.08 (1.02, 1.14) | 1.05 (0.99, 1.12) |
| HOMA-IR (%) | -- | -- | 1.14 (1.02, 1.28) | 1.12 (0.99, 1.26) |
| TNF-alpha (pg/mL) | -- | -- | 1.01 (1.00, 1.03) | 1.01 (0.99, 1.03) |
| AUC (95% CI) | 0.80 (0.75, 0.85) | 0.84 (0.78, 0.89) | 0.89 (0.86, 0.93) | 0.91 (0.87,0.94) |
| ROC curve test^{‡} | P = 0.115 | | P = 0.015 | |

| | | | | |
|---|---|---|---|---|
| *Abbreviations: EMI, body mass index; HbA1c, glycated hemoglobin; HOMA-IR, Homeostasis Model Assessment of Insulin Resistance; GP, glycome peak; ref, reference; ROC, receiver operating characteristic; te, (non-linear interaction); TNF, tumor necrosis factor.* **Odds ratio was obtained comparing the pair (GP37* = *1.5, GP24 = 3) vs. (GP37* = *1.5, GP24 = 6), ^{†}odds ratio obtained for each decimal unit increase of HbA1c, ^{‡}test comparing ROC curve discrimination capability.* | | | | |

Models 1 and 2 were univariate models that assessed the impact of HbAlc and te(GP37,GP24) on the incidence of type 2 diabetes, whereas models 3 and 4 were multivariate models that explored the diagnostic accuracy of classical risk factors alone and in combination with te(GP37,GP24).

The study of risk factors showed that HbAlc had the highest discrimination capability for predicting type 2 diabetes (AUC 0.840 [95% CI 0.78-0.89]). The interaction term te(GP37,GP24) showed the second highest AUC (0.801, 95% CI 0.75-0.85), with no significant differences between them (p=0.115) **(Table 2).** Furthermore, te(GP37,GP24) displayed a significant effect on type 2 diabetes incidence after adjusting for all risk factors, which improved the prediction accuracy of model 3 (p=0.015), reaching an AUC of 0.91 (95% CI 0.87-0.94) in model 4. Moreover, the likelihood-ratio test comparing models 3 and 4 yielded a p-value <0.001, suggesting that the identified glycome peaks have significant predictive value for the incidence of type 2 diabetes even after adjusting for risk factors **(Table** 2). Lastly, in a survival model (i.e., Cox regression), te(GP37,GP24) showed a significant effect after adjusting for the remaining risk factors **(Table 3).**

**Table 3. Effect of risk factors on type 2 diabetes incidence estimated using a Cox proportional hazard model for two univariate models (models 1 and 2) and two multivariate models (3 and 4)**

| Predictor variables | Model 1 HR (95% CI) | Model 2 HR (95% CI) | Model 3 HR (95% CI) | Model 4 HR (95% CI) |
|---|---|---|---|---|
| te(GP37,GP24)* | 1.14 (1.09, 1.18) | -- | -- | 1.06 (1.02, 1.09) |
| HbAlc (%)^{†} | -- | 1.27 (1.23, 1.32) | 1.25 (1.20, 1.31) | 1.24 (1.18, 1.30) |
| Age (years) | -- | -- | 1.01 (0.99, 1.02) | 1.01 (0.99, 1.02) |
| Sex (ref: female) | -- | -- | 1.04 (0.62, 1.76) | 1.14 (0.67, 1.94) |
| BMI (kg/m²) | -- | -- | 1.09 (1.04, 1.14) | 1.06 (1.01, 1.12) |
| HOMA-IR (%) | -- | -- | 1.12 (1.04, 1.20) | 1.09 (1.00, 1.18) |
| TNF-alpha (pg/mL) | -- | -- | 1.01 (1.00, 1.03) | 1.01 (1.00, 1.02) |
| LRT | | | P = 0.010 | |

| | | | | |
|---|---|---|---|---|
| *Abbreviations: BMI, body mass index; HbA1c, glycated hemoglobin; HOMA-IR, Homeostasis Model Assessment of Insulin Resistance; GP, glycome peak; LRT, likelihood ratio test; ref, reference; te, (non-linear interaction) term; TNF, tumor necrosis factor.* **Hazard ratio was obtained comparing the pair (GP37 = 1.5, GP24 = 3) vs (GP37 = 1.5, GP24 = 6), ^{†}hazard ratio obtained for each decimal unit increase of HbA1c.* | | | | |

## Claims

1. *In vitro* method for predicting the risk of suffering from type 2 diabetes in a subject, wherein the method comprises assessing the abundance of N-glycome peak 37 and N-glycome peak 24 in a biological sample obtained from the subject.

2. *In vitro* use of N-glycome peak 37 and N-glycome peak 24, or of a kit comprising reagents for assessing the abundance of N-glycome peak 37 and N-glycome peak 24, in a biological sample obtained from the subject, for predicting the risk of suffering from type 2 diabetes.

3. *In vitro* method, or *in vitro* use, according to any of the previous claims, wherein higher abundance of N-glycome peak 37 is an indication of a higher risk of suffering from type 2 diabetes and/or wherein higher abundance of N-glycome peak 24 is an indication of a lower risk of suffering from type 2 diabetes.

4. *In vitro* method, or *in vitro* use, according to any of the previous claims, which comprises assessing the ratio between N-glycome peak 37 and N-glycome peak 24.

5. *In vitro* method, or *in vitro* use, according to any of the previous claims, wherein the biological sample is selected from blood, plasma or serum.

6. *In vitro* method, or *in vitro* use, according to any of the previous claims, wherein the biological sample is serum.

7. *In vitro* method, or *in vitro* use, according to any of the previous claims, wherein the N-glycome peak 37 comprises triantennerary glycans containing outer arm fucose.

8. *In vitro* method, or *in vitro* use, according to any of the previous claims, wherein the N-glycome peak 37 comprises the glycans: A3F1G3S[3,3,3]3, A3F1G3S[3,3,6]3, FA3BG3S[3,3,3]3, A3G3S[3,3,6]3, *FA3BG3S[6,6,6]3, *A3G3S[3,6,6]3 and/or *A3G3S[3,3,3]3.

9. *In vitro* method, or *in vitro* use, according to any of the previous claims, wherein the N-glycome peak 24, comprises disialylated glycans.

10. *In vitro* method, or *in vitro* use, according to any of the previous claims, wherein the N-glycome peak 24, comprises the glycans: *A2G2S[3,6]2, *A2G2S[3,3]2, *FA2G2S[3,3]2, A2F1G2S[3]1 and/or A2F1G2S[6]1.

11. *In vitro* method, or *in vitro* use, according to any of the previous claims, wherein N-glycans are separated and quantified using a quantitative analytical technique.

12. *In vitro* method, or *in vitro* use, according to any of the previous claims, wherein the abundance of N-glycome peak 37 and N-glycome peak 24 is assessed by chromatography.

13. *In vitro* method, or *in vitro* use, according to any of the previous claims, wherein the abundance of N-glycome peak 37 and N-glycome peak 24 is assessed by ultra-performance liquid chromatography (UPLC), capillary electrophoresis (CE), MALDI-TOF mass spectrometry, liquid chromatography coupled with mass spectrometry (LC-MS).

14. *In vitro* method, or *in vitro* use, according to any of the previous claims, wherein the abundance of N-glycome peak 37 and N-glycome peak 24 is assessed by ultra-performance liquid chromatography (UPLC).
